# EUROPÄISCHE PATENTSCHRIFT

(11) **EP 4 527 301 B1**
(45) Veröffentlichungstag und Bekanntmachung des Hinweises auf die Patenterteilung: **29.10.2025**
(21) Anmeldenummer: 23199115.9
(22) Anmeldetag: 22.09.2023
(51) Int. Cl.: A61B 6/03, A61B 6/00

(54) **COMPUTERTOMOGRAPH MIT VERBESSERTER DATENÜBERTRAGUNG**
COMPUTED TOMOGRAPHY APPARATUS WITH IMPROVED DATA TRANSMISSION
TOMOGRAPHE ASSISTÉ PAR ORDINATEUR AVEC TRANSMISSION DE DONNÉES AMÉLIORÉE

(43) Veröffentlichungstag der Anmeldung: 26.03.2025
(73) Patentinhaber: Siemens Healthineers AG, 91301 Forchheim (DE)
(72) Erfinder: Düppenbecker, Peter Michael, 91074 Herzogenaurach (DE); Hohl, Christoph, 92256 Hahnbach (DE)
(74) Vertreter: Siemens Healthineers Patent Attorneys

(56) Entgegenhaltungen:
- EP-A1- 4 201 336
- US-A- 5 148 448
- US-A1- 2012 213 328
- US-A1- 2013 216 018

## Beschreibung

Die vorliegende Erfindung geht aus von einem Betriebsverfahren für einen Computertomographen,
- wobei eine Gantry des Computertomographen relativ zu einem feststehenden Grundkörper des Computertomographen rotiert wird,
- wobei während des Rotierens der Gantry zwischen der Gantry und dem Grundkörper digitale Daten von einer Datenquelle über einen Modulator, einen Übertragungskanal und einen Demodulator zu einer Datensinke übertragen werden.

Die vorliegende Erfindung geht weiterhin aus von einem Computertomographen,
- wobei der Computertomograph einen feststehenden Grundkörper und eine relativ zu dem feststehenden Grundkörper rotierbare Gantry aufweist,
- wobei der Computertomograph eine Datenquelle, einen Modulator, einen Übertragungskanal, einen Demodulator und eine Datensinke aufweist, so dass während des Rotierens der Gantry zwischen der Gantry und dem Grundkörper digitale Daten von der Datenquelle über den Modulator, den Übertragungskanal und den Demodulator zu der Datensinke übertragbar sind.

Computertomographen sind allgemein bekannt.

Bei einem Computertomographen sind auf der Gantry eine Röntgenquelle und ein Röntgendetektor angeordnet, wobei die Röntgenquelle während des Rotierens der Gantry Röntgenstrahlung emittiert und der Röntgendetektor die emittierte Röntgenstrahlung erfasst. Anhand der so erfassten Bilder eines Untersuchungsobjekts (meist eines Menschen, insbesondere eines Patienten) wird ein dreidimensionales Bild des Untersuchungsobjekts rekonstruiert.

Die Anmelderin weist darauf hin, dass unabhängig vom grammatikalischen Geschlecht eines bestimmten personenbezogenen Begriffs (wie hier beispielsweise des Begriffs "Patient") stets Personen mit männlicher, weiblicher und anderer Geschlechteridentität mit umfasst sind.

Das Rekonstruieren des dreidimensionalen Bildes erfolgt mittels einer Auswertungseinrichtung, die außerhalb der Gantry angeordnet ist, beispielsweise sogar außerhalb des Untersuchungsraums, in dem der Computertomograph angeordnet ist. Die mittels des Röntgendetektors erfassten Bilddaten müssen während des Rotierens von der Gantry zu der Auswertungseinrichtung übertragen werden. In der Regel erfolgt dies über einen Übertragungskanal, über den die digitalen Daten von einer Datenquelle (beispielsweise einer die erfassten Daten des Röntgendetektors geringfügig aufarbeitenden Vorauswertungseinrichtung) einem Modulator zugeführt werden, der die digitalen Daten auf ein Trägersignal aufmoduliert. Das modulierte Trägersignal wird über einen Übertragungskanal einem Demodulator zugeführt. Der Demodulator demoduliert das modulierte Trägersignal und führt das so ermittelte Empfangssignal einer Datensinke zu. Die Datenquelle und der Modulator sind in diesem Fall auf der Gantry angeordnet, der Demodulator und die Datensinke auf dem feststehenden Grundkörper. Der Übertragungskanal bildet somit die Brücke zwischen der Gantry und dem feststehenden Grundkörper.

Mit der stetigen Weiterentwicklung von Computertomographen steigt das Datenvolumen, das von der Datenquelle zu der Datensinke übertragen werden soll, immer weiter an. Konventionelle Arten der Datenübertragung stoßen zunehmend an Grenzen. Es wird daher versucht, die Datenrate, mit der die Daten übertragen werden, zu erhöhen.

Üblicherweise erfolgt bei Computertomographen eine relativ einfache Modulierung des Trägersignals, nämlich eine Phasenumtastung oder eine Amplitudenumtastung. Diese Modulierungsverfahren sind relativ robust gegenüber Phasenänderungen des übertragenen Signals. Die übertragbare Datendichte ist hingegen relativ gering. Man spricht auch von einer geringen spektralen Effizienz.

Im Stand der Technik sind Modulierungsverfahren bekannt, die eine erheblich höhere Datendichte realisieren (bzw. eine höhere spektrale Effizienz aufweisen), insbesondere die Quadraturamplitudenmodulation (QAM). Rein beispielhaft kann auf den entsprechenden Auszug aus der deutschen Wikipedia verwiesen werden, abgerufen am 21.08.2023. Bezüglich der Quadraturamplitudenmodulation existieren verschiedene "Ausbaustufen", üblicherweise als M-QAM bezeichnet, wobei M für die Anzahl an möglichen Werten steht. M ist in aller Regel eine Potenz von 2, meist sogar eine gerade Potenz von **2.** Die einfachste "Ausbaustufe" ist also eine 4-QAM. Weitere "Ausbaustufen" sind eine 16-QAM, eine 64-QAM und eine 256-QAM. Auch noch höhere "Ausbaustufen" sind bekannt.

Die Quadraturamplitudenmodulation erlaubt - bei unveränderter Bandbreite des Übertragungskanals - eine erheblich höhere Datenrate. Die Quadraturamplitudenmodulation ist jedoch sehr empfindlich gegenüber Phasenänderungen des übertragenen Signals. Denn zur korrekten Demodulierung muss auf der Demodulator die Phase des vom Modulator generierten Signals sehr gut bekannt sein. Bereits geringe Phasenverschiebungen führen dazu, dass das über den Übertragungskanal übertragene Signal nicht mehr korrekt demoduliert werden kann.

Es ist - siehe beispielsweise der bereits erwähnte Eintrag in der deutschen Wikipedia - bekannt, das über den Übertragungskanal übertragene Signal mittels einer dem Übertragungskanal nachgeordneten Nachverzerrungseinrichtung gemäß einer Nachverzerrungsvorschrift nachzuverzerren und erst das nachverzerrte Signal dem Demodulator zuzuführen. Die Nachverzerrungseinrichtung ist üblicherweise als sogenanntes Angepasstes Filter (Matched Filter) ausgebildet. Die Übertragungsfunktion dieses Filters ist auf die vom Modulator gebildeten Pulsformen ausgelegt und erlaubt eine sehr gute Störunterdrückung.

Durch die Anwendung der Quadraturamplitudenmodulation bei der Datenübertragung zwischen der Gantry und dem Grundkörper kann vom Ansatz her eine erheblich höhere Datenrate als bisher realisiert werden. Aufgrund des Umstands, dass die Datenübertragung während des Rotierens der Gantry erfolgt, verändert sich jedoch während der Datenübertragung die Laufzeit, die das vom Modulator in den Übertragungskanal eingespeiste Sendesignal benötigt, bis es den Demodulator erreicht. Damit aber ändert sich auch die Phasenbeziehung zwischen Modulator und Demodulator. Die Vorgehensweise des Standes der Technik, das über den Übertragungskanal übertragene Signal in einem Matched Filter nachzuverzerren, führt daher nicht ohne weiteres zum gewünschten Erfolg.

US 2012/213328 A1 offenbart ein Betriebsverfahren für einen Computertomographen.

Die Aufgabe der vorliegenden Erfindung besteht darin, Möglichkeiten zu schaffen, mittels derer für eine bestimmte Bandbreite des Übertragungskanals die Datenrate bei der Datenübertragung zwischen der Gantry und dem feststehenden Grundkörper erhöht werden kann.

Die Aufgabe wird durch ein Betriebsverfahren für einen Computertomographen mit den Merkmalen des Anspruchs 1 gelöst. Vorteilhafte Ausgestaltungen des Betriebsverfahrens sind Gegenstand der abhängigen Ansprüche 2 bis 8.

Erfindungsgemäß wird ein Betriebsverfahren der eingangs genannten Art dadurch ausgestaltet,
- dass ein von dem Modulator durch Modulieren eines dem Modulator von der Datenquelle zugeführten Datenstroms generiertes Sendesignal mittels einer dem Übertragungskanal vorgeordneten Vorverzerrungseinrichtung gemäß einer Vorverzerrungsvorschrift vorverzerrt wird und erst das vorverzerrte Sendesignal dem Übertragungskanal zugeführt wird und/oder ein über den Übertragungskanal übertragenes Signal mittels einer dem Übertragungskanal nachgeordneten Nachverzerrungseinrichtung gemäß einer Nachverzerrungsvorschrift nachverzerrt wird und erst das nachverzerrte Signal dem Demodulator zugeführt wird und
- dass die Vorverzerrungsvorschrift und/oder die Nachverzerrungsvorschrift von einer Einstelleinrichtung während des Rotierens der Gantry immer wieder neu eingestellt werden.

Durch die Vorverzerrung, die Nachverzerrung oder - besonders bevorzugt - die Kombination von Vorverzerrung und Nachverzerrung kann die durch die Übertragungsfunktion des Übertragungskanals bewirkte Verzerrung des Sendesignals kompensiert werden. Dem Demodulator kann somit ein im Ergebnis nahezu unverzerrtes Signal zugeführt werden, so dass eine qualitativ hochwertige, nahezu unverzerrte Rekonstruktion des eigentlichen Datensignals möglich ist. Es erfolgt also ein Angepasstes Filtern in einem angepassten Filter (Matched Filter). Insoweit entspricht die Vorgehensweise vom Ansatz her der Vorgehensweise, die im Stand der Technik für die Quadraturamplitudenmodulation ergriffen wird. Zum Kompensieren der Änderungen der Laufzeit während des Rotierens der Gantry erfolgt jedoch zusätzlich das mehr oder minder kontinuierliche Nachführen der Vorverzerrungsvorschrift und/oder der Nachverzerrungsvorschrift. Dadurch kann die Qualität der Datenübertragung auch während des Rotierens der Gantry aufrechterhalten werden.

Vorzugsweise führt die Datenquelle dem Modulator den Datenstrom als komplexes Signal zu und ermittelt der Modulator das Sendesignal durch eine Quadraturamplitudenmodulation eines Trägersignals gemäß dem im zugeführten komplexen Signal. Dadurch kann die Datenrate, mit der die Datenübertragung erfolgt, maximiert werden. Insbesondere mit einer Quadraturamplitudenmodulation zeigt die vorliegende Erfindung ihre vollen Vorteile.

Vorzugsweise sind die Vorverzerrungsvorschrift und/oder die Nachverzerrungsvorschrift parametrierbare FIR-Filterungen (FIR = Finite Impuls Response). Diese Filterungen sind zuverlässig, robust und einfach parametrierbar, auch dynamisch parametrierbar.

Vorzugsweise ermittelt die Einstelleinrichtung die Vorverzerrungsvorschrift und/oder die Nachverzerrungsvorschrift in Abhängigkeit von einem jeweiligen Drehwinkel der Gantry.

Vorzugsweise berücksichtigt die Einstelleinrichtung im Rahmen der Ermittlung der Vorverzerrungsvorschrift und/oder der Nachverzerrungsvorschrift zusätzlich zu dem jeweiligen Drehwinkel mindestens eine zeitliche Ableitung des Drehwinkels.

Bei den zeitlichen Ableitungen kann es sich insbesondere um die erste zeitliche Ableitung (das heißt die Drehgeschwindigkeit) und/oder die zweite zeitliche Ableitung (d.h. die Drehbeschleunigung) handeln. Die zeitlichen Ableitungen werden insbesondere vorzeichenrichtig berücksichtigt. Durch die Berücksichtigung der zeitlichen Ableitungen des Drehwinkels können beispielsweise zeitliche Verzögerungen zwischen dem Erfassen oder Ermitteln des jeweiligen Drehwinkels und dem Nachführen der Vorverzerrungsvorschrift und/oder der Nachverzerrungsvorschrift kompensiert werden.

Es ist möglich, dass der Drehwinkel jeweils gemessen wird, dass also dem Nachführen der Vorverzerrungsvorschrift und/ oder der Nachverzerrungsvorschrift jeweils ein gemessener Drehwinkel zu Grunde liegt. Auch in diesem Fall ist es möglich, im Rahmen der Ermittlung der Vorverzerrungsvorschrift und/oder der Nachverzerrungsvorschrift zusätzlich auch mindestens eine zeitliche Ableitung des Drehwinkels mit zu berücksichtigen.

Alternativ ist es möglich, dass der Drehwinkel nur bei vorbestimmten Winkelpositionen gemessen wird, beispielsweise nur alle 120°, alle 180° oder sogar nur einmal pro vollständiger Umdrehung der Gantry. In diesem Fall wird der Drehwinkel zwischen den vorbestimmten Winkelpositionen von der Einstelleinrichtung durch Fortschreibung des zuletzt gemessenen Drehwinkels anhand von die Drehung der Gantry charakterisierenden Betriebsdaten der Gantry, insbesondere der Drehzahl und/oder der Drehbeschleunigung, ermittelt.

Vorzugsweise sind in der Einstelleinrichtung in einer Lookup-Table für eine Vielzahl von Eingangsgrößen jeweils Parameter für die Parametrierung der Vorverzerrungseinrichtung und/oder der Nachverzerrungseinrichtung hinterlegt und ermittelt die Einstelleinrichtung die Parameter unter Verwertung der Lookup-Table.

Beispielsweise kann die Lookup-Table für eine Vielzahl von Stützstellen des Drehwinkels jeweils die zugehörigen Parameter zur Parametrierung der Vorverzerrungseinrichtung und/oder der Nachverzerrungseinrichtung enthalten. Stimmt der jeweilige Drehwinkel mit einer der Stützstellen überein, so werden die für diese Stützstelle hinterlegten Parameter verwendet. Stimmt der jeweilige Drehwinkel mit keiner der Stützstellen überein, so können die Parameter für den jeweiligen Drehwinkel beispielsweise mittels einer linearen Interpolation der für die nächstliegenden Stützstellen hinterlegten Parameter ermittelt werden. Ähnliche Vorgehensweisen sind möglich, wenn die Lookup-Table nicht eindimensional (Verwertung nur des Drehwinkels), sondern mehrdimensional (Verwertung zusätzlich auch der Drehgeschwindigkeit usw.) ist.

Vorzugsweise umfasst der Datenstrom eine Vielzahl von Datengruppen, wobei die Datengruppen jeweils eine Referenzsequenz und eine Nutzsequenz umfassen. In diesem Fall umfasst das über den Übertragungskanal übertragene Signal einen Referenzanteil und einen Nutzanteil. Die Referenzanteile werden in diesem Fall der Einstelleinrichtung zugeführt und die Einstelleinrichtung ermittelt die Nachverzerrungsvorschrift für den auf den jeweiligen Referenzanteil folgenden Nutzanteil in Abhängigkeit von dem jeweiligen Referenzanteil. Hintergrund dieser Vorgehensweise ist, dass die Referenzsequenz der Einstelleinrichtung vorab bekannt sein kann, so dass der Einstelleinrichtung bekannt sein kann, dass die sich nach dem Nachverzerren und dem Demodulieren ergebende rekonstruierte Sequenz mit der Referenzsequenz übereinstimmen müsste. Die Einstelleinrichtung kann daher die Nachverzerrungsvorschrift derart nachführen, dass die rekonstruierte Sequenz möglichst gut mit der Referenzsequenz übereinstimmt, und für das Nachverzerren des Nutzanteils der entsprechenden Datengruppe die entsprechend nachgeführte Nachverzerrungsvorschrift verwenden. Bei dem übertragenen Signal kann es sich um bereits das nachverzerrte übertragene Signal oder um das noch nicht nachverzerrte übertragene Signal und auch um aus diesen Signalen abgeleitete Daten handeln.

Die Aufgabe wird weiterhin durch einen Computertomographen mit den Merkmalen des Anspruchs 9 gelöst. Vorteilhafte Ausgestaltungen des Computertomographen sind Gegenstand der abhängigen Ansprüche 10 bis 15.

Erfindungsgemäß wird ein Computertomograph der eingangs genannten Art dadurch ausgestaltet,
- dass dem Übertragungskanal eine Vorverzerrungseinrichtung vorgeordnet ist, mittels derer ein von dem Modulator durch Modulieren eines dem Modulator von der Datenquelle zugeführten Datenstroms generiertes Sendesignal gemäß einer Vorverzerrungsvorschrift vorverzerrt wird, so dass erst das vorverzerrte Sendesignal dem Übertragungskanal zugeführt wird, und/oder dem Übertragungskanal eine Nachverzerrungseinrichtung nachgeordnet ist, mittels derer ein über den Übertragungskanal übertragenes Signal gemäß einer Nachverzerrungsvorschrift nachverzerrt wird, so dass erst das nachverzerrte Signal dem Demodulator zugeführt wird, und
- dass der Computertomograph eine Einstelleinrichtung aufweist, von der die Vorverzerrungsvorschrift und/oder die Nachverzerrungvorschrift während des Rotierens der Gantry immer wieder neu eingestellt werden.

Die dadurch bewirkten Sachverhalte und Vorteile korrespondieren mit denen des erfindungsgemäßen Betriebsverfahrens.

Die vorteilhaften Ausgestaltungen des Computertomographen korrespondieren mit den vorteilhaften Ausgestaltungen des Betriebsverfahrens. Gleiches gilt für die dadurch bewirkten Vorteile.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: einen Computertomographen,
- FIG 2: ein Zeitdiagramm,
- FIG 3: ein weiteres Zeitdiagramm,
- FIG 4: eine Datenübertragungsstruktur,
- FIG 5: einen Kommunikationsweg,
- FIG 6: ein Ablaufdiagramm,
- FIG 7: eine Einstelleinrichtung,
- FIG 8: eine Datenquelle und einen Modulator,
- FIG 9: einen Demodulator und eine Datensinke,
- FIG 10: einen Datenstrom,
- FIG 11: ein übertragenes Signal und
- FIG 12: ein Ablaufdiagramm.

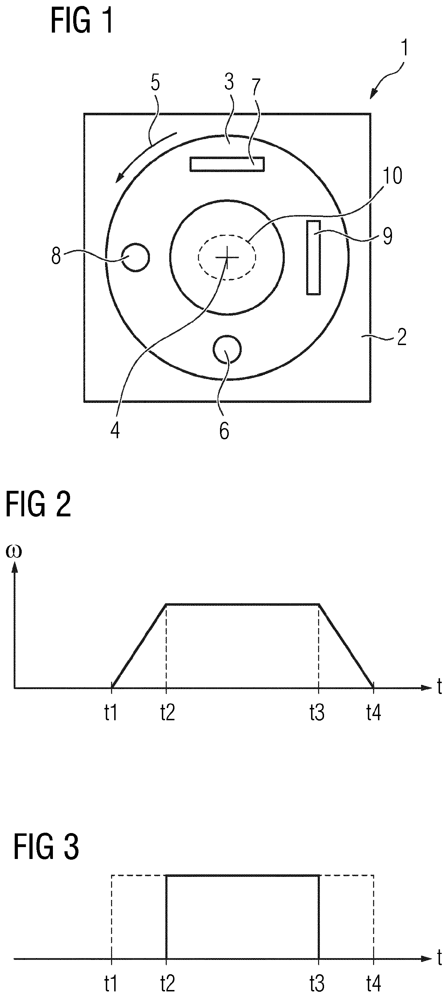

Gemäß FIG 1 weist ein Computertomograph 1 einen feststehenden Grundkörper 2 auf. An dem Grundkörper 2 ist eine Gantry 3 drehbar gelagert, so dass die Gantry 3 relativ zum Grundkörper 2 um eine Rotationsachse 4 rotierbar ist. Die Rotierbarkeit ist in FIG 1 durch einen Pfeil 5 angedeutet. Die Gantry 3 trägt, wie allgemein üblich, eine Röntgenquelle 6 und einen Röntgendetektor 7. Die Röntgenquelle 6 und der Röntgendetektor 7 liegen einander bezüglich der Rotationsachse 4 diametral gegenüber. In manchen Fällen trägt die Gantry 3 zusätzlich auch eine weitere Röntgenquelle 8 und einen weiteren Röntgendetektor 9. Auch die weitere Röntgenquelle 8 und der weitere Röntgendetektor 9 liegen einander bezüglich der Rotationsachse 4 diametral gegenüber. Die weitere Röntgenquelle 8 und der weitere Röntgendetektor 9 sind, sofern sie vorhanden sind, in aller Regel bezüglich der erstgenannten Röntgenquelle 6 und dem erstgenannten Röntgendetektor 7 um 90° in Umfangsrichtung um die Rotationsachse 4 herum gesehen versetzt angeordnet. Während des Rotierens der Gantry 3 um die Rotationsachse 4 können mittels des Röntgendetektors 7 bzw. der Röntgendetektoren 7, 9 Röntgenbilder eines Untersuchungsobjekts 10 erfasst werden, das im Bereich der Rotationsachse 4 angeordnet ist.

FIG 2 zeigt das Rotieren der Gantry 3 als Funktion der Zeit t. Gemäß FIG 2 wird zum Erfassen der Röntgenbilder des Untersuchungsobjekts 10 zu einem Zeitpunkt t1 mit dem Rotieren der Gantry 3 begonnen. Konkret wird die Gantry 3 ab dem Zeitpunkt t1 bis zu einem Zeitpunkt t2 beschleunigt, so dass eine Drehgeschwindigkeit ω zum Zeitpunkt t2 einen Maximalwert erreicht. Danach rotiert die Gantry 3 bis zu einem Zeitpunkt t3 mit der maximalen Drehgeschwindigkeit und wird schließlich wieder abgebremst, bis sie zu einem Zeitpunkt t4 zum Stillstand kommt.

Das Erfassen der Röntgenbilder erfolgt zumindest zwischen den Zeitpunkten t2 und t3, oftmals sogar ab dem Zeitpunkt t1 und bis zum Zeitpunkt t4. Mehr oder minder simultan zum Erfassen der Röntgenbilder werden die Röntgenbilder gemäß FIG 3 auch von der Gantry 3 an den Grundkörper 2 übertragen. FIG 3 zeigt in einer durchgezogenen Linie den Zeitraum, während dessen zumindest die Datenübertragung erfolgt, und in gestrichelten Linien die Zeiträume, während derer ebenfalls eine Datenübertragung erfolgen kann. Die übertragenen Daten sind digitaler Natur.

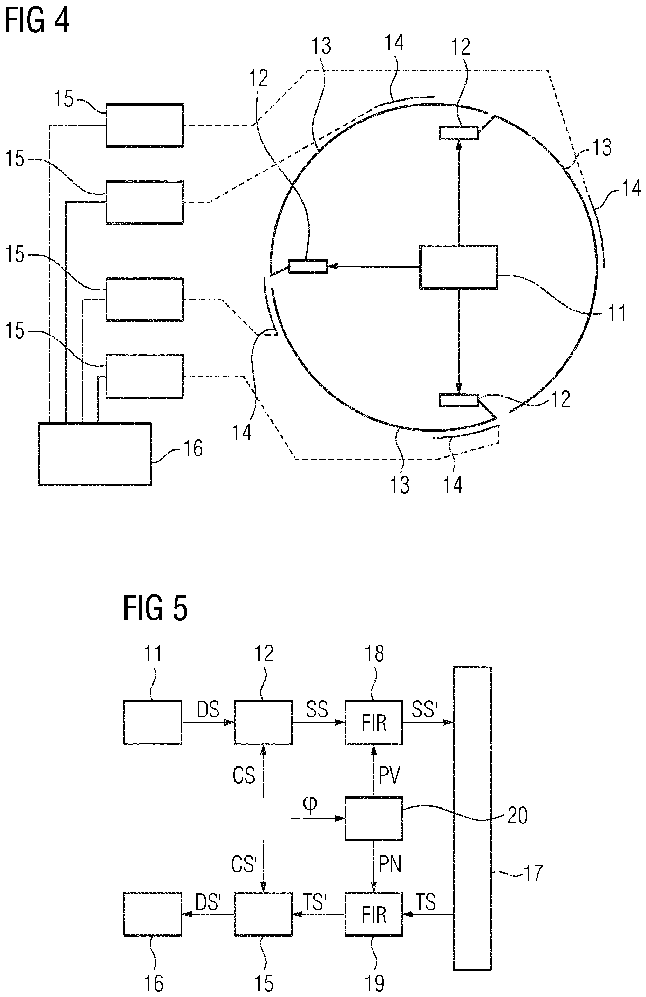

FIG 4 zeigt in vereinfachter Form eine Struktur zur Übertragung der Daten zwischen der Gantry 3 und dem Grundkörper 2.

Gemäß FIG 4 ist eine Datenquelle 11 vorhanden. Die Datenquelle 11 kann beispielsweise eine Einheit sein, welche von den Röntgendetektoren 7, 9 deren Bilder entgegennimmt und sie (geringfügig) aufbereitet. Die Datenquelle 11 ist mit einer Anzahl an Modulatoren 12 verbunden. Minimal ist ein einziger Modulator 12 vorhanden. Vorliegend sind drei Modulatoren 12 vorhanden. Die Modulatoren 12 speisen von ihnen generierte Sendesignale SS' (siehe FIG 5) in gantryseitige Abschnitte 13 von Übertragungskanälen 17 (siehe ebenfalls FIG 5) ein. Die Abschnitte 13 erstrecken sich jeweils über (knapp) 360°/n, wobei n die Anzahl an Modulatoren 12 ist. Die gantryseitigen Abschnitte 13 können beispielsweise als Wellenleiter oder als dielektrische Leiter ausgebildet sein. An die gantryseitigen Abschnitte 13 sind auf Seiten des feststehenden Grundkörpers 2 Koppelelemente 14 angekoppelt. Die Koppelelemente 14 sind in der Regel gleichmäßig über den Umfang verteilt. Die Anzahl an Koppelelementen 14 ist meistens um 1 größer als die Anzahl an Modulatoren 12. Von den Koppelelementen 14 aus werden die Signale weiter an Demodulatoren 15 von und von dort aus weiter an eine Datensinke 16 übermittelt. Zwischen den Koppelelementen 14 und den Demodulatoren 15 besteht in aller Regel eine 1:1-Beziehung. Der Abschnitt von einem der Modulatoren 12 bis zu einem der Demodulatoren 15 bildet jeweils einen Übertragungskanal 17.

Die Datenübertragung wurde vorstehend in Verbindung mit einer Übertragung von Daten von der Gantry 3 zum Grundkörper 2 erläutert. Prinzipiell könnte die Datenübertragung aber auch in der umgekehrten Richtung erfolgen. Weiterhin könnten auch umgekehrt die Abschnitte 13 auf Seiten des Festkörpers 2 und die Koppelelemente 14 auf Seiten der Gantry 3 angeordnet sein.

Nachfolgend wird in Verbindung mit FIG 5 der Kommunikationsweg von der Datenquelle 11 zur Datensinke 16 für einen einzelnen Übertragungsweg nochmals erläutert.

Gemäß FIG 5 werden die Daten in Form eines Datenstroms DS von der Datenquelle 11 über einen der Modulatoren 12, den zugehörigen Übertragungskanal 17 und einen der Demodulatoren 15 zur Datensinke 16 übertragen. Die Datenübertragung erfolgt - vergleiche die FIG 2 und 3 während des Rotierens der Gantry 3. Konkret übermittelt die Datenquelle 11 an den entsprechenden Modulator 12 den Datenstrom DS. Der Modulator 12 moduliert gemäß dem Datenstrom DS ein Trägersignal CS. Das modulierte Trägersignal entspricht einem Sendesignal SS, das der Modulator 12 vom Ansatz her in den Übertragungskanal 17 einspeisen soll. Das Sendesignal SS wird jedoch zunächst einer Vorverzerrungseinrichtung 18 zugeführt. Die Vorverzerrungseinrichtung 18 ist mit Parametern PV parametriert. Die Parametrierung der Vorverzerrungseinrichtung 18 mit den Parametern PV legt eine Vorverzerrungsvorschrift fest, gemäß der die Vorverzerrungseinrichtung 18 eine Vorverzerrung des Sendesignals SS vornimmt. Erst das vorverzerrte Sendesignal - nachfolgend zur Unterscheidung von dem ursprünglichen Sendesignal SS mit dem Bezugszeichen SS' versehen - wird dem Übertragungskanal 17 zugeführt und somit in den Übertragungskanal 17 eingespeist. Im Ergebnis ist somit die Vorverzerrungseinrichtung 18 dem Übertragungskanal 17 vorgeordnet.

Das in den Übertragungskanal 17 eingespeiste Signal SS' wird über den Übertragungskanal 17 übertragen. Dieses Signal wird nachstehend als übertragenes Signal bezeichnet und ist mit dem Bezugszeichen TS versehen. Nach dem Übertragen wird das übertragene Signal TS einer Nachverzerrungseinrichtung 19 zugeführt. Die Nachverzerrungseinrichtung 19 ist mit Parametern PN parametriert. Die Parametrierung der Nachverzerrungseinrichtung 19 mit den Parametern PN legt eine Nachverzerrungsvorschrift fest, gemäß der die Nachverzerrungseinrichtung 19 eine Nachverzerrung des übertragenen Signals TS vornimmt. Erst das nachverzerrte übertragene Signal - nachfolgend zur Unterscheidung von dem übertragenen Signal TS mit dem Bezugszeichen TS' versehen - wird dem Demodulator 15 zugeführt. Im Ergebnis ist somit die Nachverzerrungseinrichtung 19 dem Übertragungskanal 17 nachgeordnet.

Der Demodulator 15 demoduliert das nachverzerrte übertragene Signal TS' und generiert dadurch einen übertragenen Datenstrom DS'. Der übertragene Datenstrom DS' wird der Datensinke 16 zugeführt.

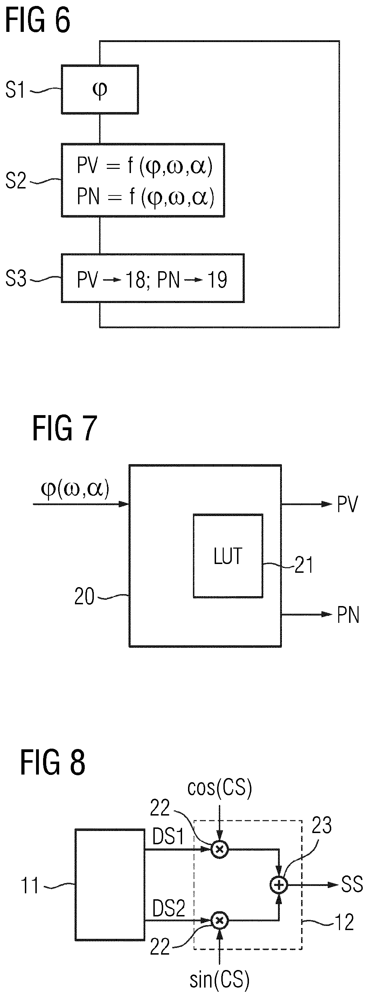

Wie aus FIG 4 ersichtlich ist, ändert sich während des Rotierens der Gantry 3 laufend der Bereich des jeweiligen Abschnitts 13, an dem das jeweils angrenzende Koppelelement 14 mit dem jeweiligen Abschnitt 13 koppelt. Demzufolge ändert sich während des Rotierens der Gantry 3 laufend die effektive Länge des Übertragungskanals 17. Demzufolge ändern sich auch die Übertragungseigenschaften des Übertragungskanals 17. Um die Übertragungseigenschaften der Gesamtheit von Übertragungskanal 17, Vorverzerrungseinrichtung 18 und Nachverzerrungseinrichtung 19 konstant bzw. zumindest im Wesentlichen konstant zu halten, ist eine Einstelleinrichtung 20 vorhanden. Die Einstelleinrichtung 20 stellt während des Rotierens der Gantry 3 durch Vorgabe der entsprechenden Parameter PV, PN die Vorverzerrungsvorschrift und/oder die Nachverzerrungsvorschrift immer wieder neu ein. Dies wird nachstehend in Verbindung mit FIG 6 näher erläutert.

Gemäß FIG 6 wird der Einstelleinrichtung 20 in einem Schritt S1 ein jeweiliger Drehwinkel φ der Gantry 3 bekannt. Es ist möglich, dass der Drehwinkel φ messtechnisch erfasst wird, also gemessen wird, und der entsprechende Messwert φ der Einstelleinrichtung 20 zugeführt wird. Alternativ ist es möglich, dass der zugehörige Drehwinkel φ nur bei vorbestimmten Winkelpositionen der Gantry 3 - beispielsweise jeweils bei Passieren einer Referenzposition - direkt oder indirekt gemessen wird und zwischen den vorbestimmten Winkelpositionen eine Fortschreibung des zuletzt gemessenen Drehwinkels φ erfolgt. Die Fortschreibung kann insbesondere anhand von die Drehung der Gantry 3 charakterisierenden Betriebsdaten ω, α der Gantry (3) erfolgen, beispielsweise der Drehzahl ω und/ oder der Drehbeschleunigung α.

In einem Schritt S2 ermittelt die Einstelleinrichtung 20 die Parameter PV, PN für die Vorverzerrungseinrichtung 18 und die Nachverzerrungseinrichtung 19. Die Ermittlung erfolgt in Abhängigkeit von dem jeweiligen Drehwinkel φ. Gegebenenfalls kann, wie in FIG 6 angedeutet, zusätzlich auch mindestens eine zeitliche Ableitung des Drehwinkels φ mit berücksichtigt werden, insbesondere die erste zeitliche Ableitung, d.h. die Drehgeschwindigkeit ω, und gegebenenfalls auch die zweite zeitliche Ableitung, d.h. die Drehbeschleunigung α.

In einem Schritt S3 gibt die Einstelleinrichtung 20 die im Schritt S2 ermittelten Parameter PV, PN an die Vorverzerrungseinrichtung 18 und die Nachverzerrungseinrichtung 19 aus. Dadurch erfolgt die Neueinstellung der entsprechenden Verzerrungsvorschriften.

Sodann geht die Einstelleinrichtung 20 zum Schritt S1 zurück, so dass sie im Ergebnis die Schritte S1 bis S3 kontinuierlich immer wieder ausführt.

Die vorstehenden Erläuterungen sind in gewissem Umfang vereinfacht. So ist es insbesondere nicht erforderlich, nach dem Schritt S3 sofort wieder den Schritt S1 auszuführen. In vielen Fällen reicht es aus, die im Schritt S3 vorgenommene jeweilige Festlegung der Parameter PV, PN beizubehalten, bis eine bestimmte Zeit verstrichen ist oder die Gantry 3 sich um einen bestimmten Winkel weiter gedreht hat.

Nachstehend wird das Grundprinzip zur Bestimmung der Parameter PV, PN erläutert.

Wenn mit H, U und V die Übertragungsfunktionen des Übertragungskanals 17, der Vorverzerrungseinrichtung 18 und der Nachverzerrungseinrichtung 19 bezeichnet werden, so gilt stets H' = UHV, wobei H' die resultierende Übertragungsfunktion von Vorverzerrungseinrichtung 18, Übertragungskanal 17 und Nachverzerrungseinrichtung 19 ist. Idealerweise sollte weiterhin die Beziehung H' = I gelten, wobei I die Einheits-Übertragungsfunktion ist.

Die Übertragungsfunktion H des Übertragungskanals 17 ist aufgrund der Rotation der Gantry 3 zeitlich variabel. Die Parameter PV, PN, mit denen die Vorverzerrungseinrichtung 18 und die Nachverzerrungseinrichtung 19 parametriert werden und die somit die entsprechenden Verzerrungsvorschriften und damit deren Übertragungsfunktionen U, V festlegen, sollten also jederzeit derart bestimmt werden, dass die genannte Beziehung H' = I exakt oder zumindest näherungsweise gilt.

Je nach Sachlage kann es ausreichend sein, wenn nur entweder die Vorverzerrungseinrichtung 18 vorhanden ist oder die Nachverzerrungseinrichtung 19 vorhanden ist oder zwar beide Verzerrungseinrichtungen 18, 19 vorhanden sind, aber nur die Parameter PV, PN einer der beiden Verzerrungseinrichtungen 18, 19 immer wieder neu eingestellt werden. In aller Regel ist es jedoch vorzuziehen, wenn sowohl die Vorverzerrungseinrichtung 18 als auch die Nachverzerrungseinrichtung 19 vorhanden sind und auch die Parameter PV, PN, beider Verzerrungseinrichtungen 18, 19 immer wieder neu eingestellt werden.

Die Ausgestaltung der Vorverzerrungseinrichtung 18 und der Nachverzerrungseinrichtung 19 kann nach Bedarf sein. In vielen Fällen sind die beiden Verzerrungseinrichtungen 18, 19 entsprechend der Darstellung in FIG 5 als parametrierbare FIR-Filter ausgebildet bzw. ist im Falle nur einer Verzerrungseinrichtung 18 oder 19 die vorhandene Verzerrungseinrichtung 18, 19 als parametrierbares FIR-Filter ausgebildet. Die Parametrierung des jeweiligen Filters ergibt sich durch die jeweiligen Parameter PV, PN. Die Vorverzerrungsvorschrift und/oder die Nachverzerrungsvorschrift sind somit im Ergebnis vorzugsweise parametrierbare FIR-Filterungen.

Auch die Ausgestaltung der Einstelleinrichtung 20 kann nach Bedarf sein. So ist es beispielsweise möglich, dass die Einstelleinrichtung 20 die Parameter PV, PN unter Verwertung des Drehwinkels φ (gegebenenfalls mit zusätzlicher Verwertung der Drehzahl ω und/oder der Drehbeschleunigung α) rechnerisch ermittelt. Erheblich einfacher ist jedoch eine Ausgestaltung, wie sie nachstehend in Verbindung mit FIG 7 erläutert wird.

Gemäß FIG 7 weist die Einstelleinrichtung 20 eine Lookup-Table 21 auf. In der Lookup-Table 21 sind für eine Vielzahl von Eingangsgrößen jeweils Parameter für die Parametrierung der Vorverzerrungseinrichtung 18 und/oder der Nachverzerrungseinrichtung 19 hinterlegt. Im einfachsten Fall ist die einzige Eingangsgröße der Lookup-Table 21 der Drehwinkel φ. In diesem eindimensionalen Fall können beispielsweise für Werte von 0° bis 360° in einem Raster von 2° die zugehörigen Parameter PV, PN für die beiden Verzerrungseinrichtungen 18, 19 hinterlegt sein. Das Rastermaß kann natürlich auch feiner oder gröber sein. Im Falle mehrerer Eingangsgrößen - beispielsweise des Drehwinkels φ und der Winkelgeschwindigkeit ω - ist die Lookup-Table 21 mehrdimensional. In diesem Fall gelten für die Winkelgeschwindigkeit ω analoge Ausführungen. Gleiches gilt bei einer (gegebenenfalls zusätzlichen) Erweiterung um die Winkelbeschleunigung α als weitere Eingangsgröße der Lookup-Table 21. Im Falle einer Lookup-Table 21 kann die Einstelleinrichtung 20 die Parameter PV, PN unter Verwertung der Lookup-Table 21 ermitteln. Die Art und Weise der Verwendung einer Lookup-Table 21 ist allgemein bekannt und muss daher nicht näher erläutert werden.

Die Datenquelle 11 führt entsprechend der Darstellung in FIG 8 den Datenstrom DS dem Modulator 12 vorzugsweise als komplexes Signal zu. Der Datenstrom DS umfasst somit zwei Teilsignale DS1, DS2, die den Realteil und den Imaginärteil des komplexen Signals darstellen. Der Modulator 12 ist in diesem Fall als Quadraturamplitudenmodulator ausgebildet. Er umfasst zwei Multiplizierer 22, denen der Kosinus und der Sinus des Trägersignals CS zugeführt werden. Den beiden Multiplizierern 22 wird weiterhin jeweils eines der beiden Teilsignale DS1, DS2 zugeführt. Die von den beiden Multiplizierern 22 generierten Signale werden einem Addierer 23 zugeführt, der die beiden Signale zu dem Sendesignal SS addiert.

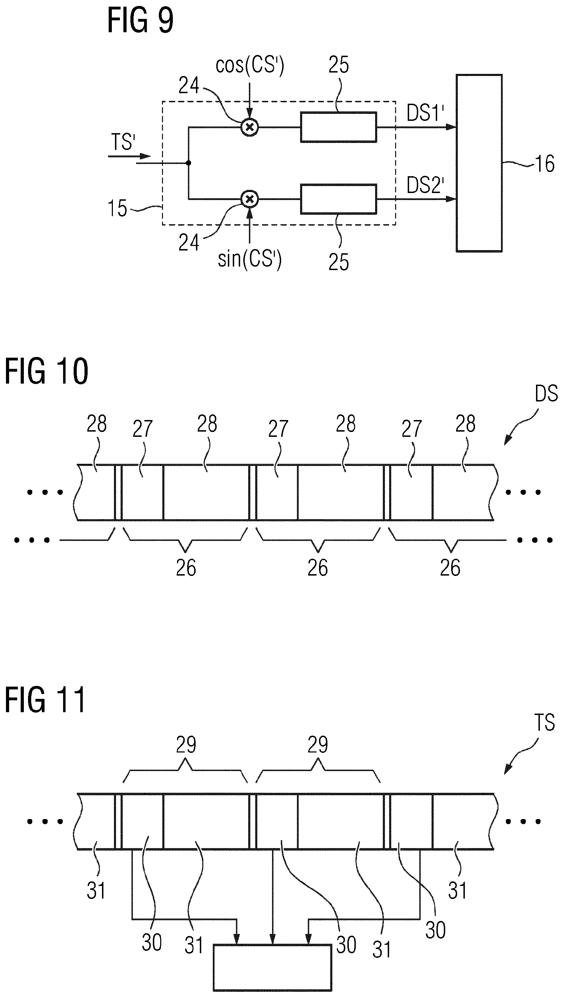

Im Falle einer Quadraturamplitudenmodulation sind gemäß der Darstellung in FIG 9 auch der Demodulator 15 und die Datensinke 16 entsprechend ausgebildet. Insbesondere umfasst in diesem Fall der Demodulator 15 zwei Multiplizierer 24, denen zum einen das nachverzerrte übertragene Signal TS' und der Kosinus und der Sinus eines mit dem Trägersignal CS frequenzgleichen weiteren Trägersignals CS' zugeführt werden. Die beiden Multiplizierer 24 liefern als Ausgangssignale den Realteil DS1' und den Imaginärteil DS2' des demodulierten Signals DS', denen ein jeweiliger hochfrequenter Anteil überlagert ist. Der jeweilige hochfrequente Anteil wird in einem jeweiligen Tiefpassfilter 25 ausgefiltert, so dass am Ausgang des jeweiligen Tiefpassfilters 25 der Realteil DS1' und der Imaginärteil DS2' des demodulierten Signals DS' zur Verfügung stehen. Diese beiden Teilsignale DS1', DS2' werden der Datensinke 16 zugeführt.

Zur korrekten Demodulation muss die Phase des weiteren Trägersignals CS' mit der Phase des Trägersignals CS übereinstimmen. Insbesondere diese Phasenübereinstimmung wird durch die beiden Verzerrungseinrichtungen 18, 19 und die dynamische Einstellung von deren Verzerrungsfunktionen bewirkt.

Gemäß FIG 10 kann der Datenstrom DS eine Vielzahl von Datengruppen 26 umfassen. Dies gilt auch und ganz besonders in dem Fall, dass der Datenstrom DS ein komplexer Datenstrom ist. Die Datengruppen 26 umfassen jeweils eine Referenzsequenz 27 und eine Nutzsequenz 28. Die Referenzsequenz 27 steht vorab fest. Ihr Inhalt ist also nicht nur der Datenquelle 11, sondern auch der Datensinke 16 bekannt. Die Nutzsequenz 28 enthält die eigentlich zu übertragenden Nutzdaten, also beispielsweise die Daten der Röntgenbilder. Die Datengruppen 26 werden sequenziell nacheinander übermittelt, wobei jeweils zuerst die jeweilige Referenzsequenz 27 und erst danach die jeweilige Nutzsequenz 28 übermittelt wird.

Entsprechend der Abfolge von Datengruppen 26 des Datenstroms DS und deren Aufbau umfasst das über den Übertragungskanal 17 übertragene Signal TS gemäß FIG 11 Abschnitte 29, die jeweils einen Referenzanteil 30 und einen Nutzanteil 31 umfassen. Die Abschnitte 29 korrespondieren jeweils mit einer Datengruppe 26, die Referenzanteile 30 mit der jeweils zugehörigen Referenzsequenz 27 und die Nutzanteile 31 mit der jeweils zugehörigen Nutzsequenz 28. Gleiches gilt - dies ist nicht eigens dargestellt - auch für das nachverzerrte übertragene Signal TS'.

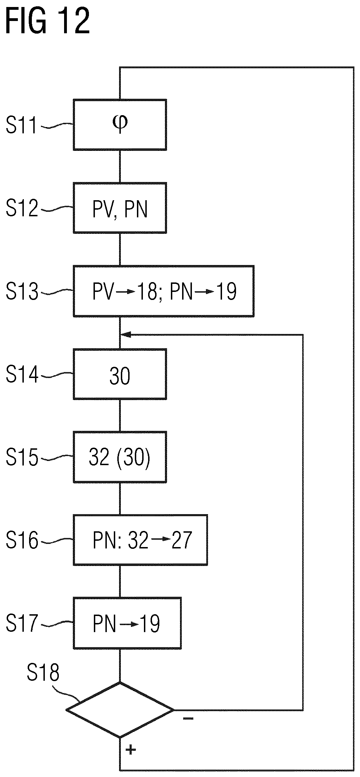

Die Referenzanteile 30 können entsprechend der Darstellung in FIG 11 der Einstelleinrichtung 20 zugeführt werden. In diesem Fall kann die vorstehend erläuterte Parametrierung der Nachverzerrungseinrichtung 19 - nicht der Vorverzerrungseinrichtung 18 - derart modifiziert werden, wie dies nachfolgend in Verbindung mit FIG 12 erläutert wird.

Gemäß FIG 12 wird der Einstelleinrichtung 20 in einem Schritt S11 der jeweilige Drehwinkel φ der Gantry 3 bekannt. In einem Schritt S12 ermittelt die Einstelleinrichtung 20 die Parameter PV, PN für die Vorverzerrungseinrichtung 18 und die Nachverzerrungseinrichtung 19. In einem Schritt S13 gibt die Einstelleinrichtung 20 die ermittelten Parameter PV, PN an die Vorverzerrungseinrichtung 18 und die Nachverzerrungseinrichtung 19 aus. Die Schritte S11 bis S13 korrespondieren 1:1 mit den Schritten S1 bis S3 von FIG 6. Es wird daher auf die zugehörigen Ausführungen verwiesen.

In einem Schritt S14 nimmt die Einstelleinrichtung 20 einen jeweiligen Referenzanteil 30 entgegen. In einem Schritt S15 ermittelt die Einstelleinrichtung 20 durch Demodulieren des Referenzanteils 30 eine zugehörige rekonstruierte Sequenz 32. In einem Schritt S16 korrigiert die Einstelleinrichtung 20 die Parameter PN für die Nachverzerrungseinrichtung 19. Die Korrektur erfolgt mit dem Ziel, die rekonstruierte Sequenz 32 so weit wie möglich an die Referenzsequenz 27 anzunähern, im Idealfall mit ihr in völlige Übereinstimmung zu bringen. Soweit erforderlich, können die Schritte S15 und S16 iterativ ausgeführt werden, bis die Parameter PN vollständig optimiert oder zumindest in hinreichendem Umfang optimiert sind. In einem Schritt S17 gibt die Einstelleinrichtung 20 die korrigierten Parameter PN an die Nachverzerrungseinrichtung 19 aus. Im Ergebnis wird durch die Schritte S14 bis S17 somit bewirkt, dass die Einstelleinrichtung 20 die Nachverzerrungsvorschrift in Abhängigkeit von dem jeweiligen Referenzanteil 30 nachführt. Gemäß der so korrigierten Nachverzerrungsvorschrift wird mittels des Demodulators 15 der zugehörige Nutzanteil 31 demoduliert.

In einem Schritt S18 prüft die Einstelleinrichtung 20, ob eine Neubestimmung der Parameter PV für die Vorverzerrungseinrichtung 18 erforderlich ist. Die Prüfung kann beispielsweise ein Zeitablauf oder das Überstreichen eines bestimmten Winkelbereichs oder das Überschreiten eines vorbestimmten Winkels umfassen. Ergibt die Prüfung des Schrittes S18, dass eine Neubestimmung der Parameter PV für die Vorverzerrungseinrichtung 18 nicht erforderlich ist, so geht die Einstelleinrichtung 20 zum Schritt S14 zurück, so dass sie im Ergebnis die Schritte S14 bis S17 mehrmals hintereinander ausführt. Ergibt die Prüfung des Schrittes S18 hingegen, dass eine Neubestimmung der Parameter PV für die Vorverzerrungseinrichtung 18 erforderlich ist, so geht die Einstelleinrichtung 20 zum Schritt S11 zurück, so dass die Schritte S11 bis S13 erneut ausgeführt werden.

Die Vorgehensweise von FIG 12 wurde basierend auf dem übertragenen Signal TS erläutert, also dem noch nicht nachverzerrten übertragenen Signal. Es ist jedoch ebenso möglich, die Vorgehensweise von FIG 12 mit dem nachverzerrten übertragenen Signals TS' durchzuführen. In diesem Fall kann der Schritt S15 entfallen.

Zusammengefasst betrifft die vorliegende Erfindung somit folgenden Sachverhalt:
Eine Gantry 3 eines Computertomographen wird relativ zu einem feststehenden Grundkörper 2 des Computertomographen rotiert.

Während des Rotierens der Gantry 3 werden zwischen der Gantry 3 und dem Grundkörper 2 digitale Daten von einer Datenquelle 11 über einen Modulator 12, einen Übertragungskanal 17 und einen Demodulator 15 zu einer Datensinke 16 übertragen. Ein von dem Modulator 12 durch Modulieren eines dem Modulator 12 von der Datenquelle 11 zugeführten Datenstroms DS generiertes Sendesignal SS wird mittels einer dem Übertragungskanal 17 vorgeordneten Vorverzerrungseinrichtung 18 gemäß einer Vorverzerrungsvorschrift vorverzerrt. Erst das vorverzerrte Sendesignal SS' wird dem Übertragungskanal 17 zugeführt. Alternativ oder zusätzlich wird ein über den Übertragungskanal 17 übertragenes Signal TS mittels einer dem Übertragungskanal 17 nachgeordneten Nachverzerrungseinrichtung 19 gemäß einer Nachverzerrungsvorschrift nachverzerrt und wird erst das nachverzerrte Signal TS' dem Demodulator 15 zugeführt. Die Vorverzerrungsvorschrift und/oder die Nachverzerrungsvorschrift werden von einer Einstelleinrichtung 20 während des Rotierens der Gantry 3 immer wieder neu eingestellt.

Die vorliegende Erfindung weist viele Vorteile auf. Insbesondere wird eine vergleichsweise einfache und dennoch robuste Datenübertragung geschaffen, mittels derer hohe Übertragungsraten im Bereich von etlichen Gigabit pro Sekunde realisiert werden können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Betriebsverfahren für einen Computertomographen,
- wobei eine Gantry (3) des Computertomographen relativ zu einem feststehenden Grundkörper (2) des Computertomographen rotiert wird,
- wobei während des Rotierens der Gantry (3) zwischen der Gantry (3) und dem Grundkörper (2) digitale Daten von einer Datenquelle (11) über einen Modulator (12), einen Übertragungskanal (17) und einen Demodulator (15) zu einer Datensinke (16) übertragen werden,
- wobei ein von dem Modulator (12) durch Modulieren eines dem Modulator (12) von der Datenquelle (11) zugeführten Datenstroms (DS) generiertes Sendesignal (SS) mittels einer dem Übertragungskanal (17) vorgeordneten Vorverzerrungseinrichtung (18) gemäß einer Vorverzerrungsvorschrift vorverzerrt wird und erst das vorverzerrte Sendesignal (SS') dem Übertragungskanal (17) zugeführt wird und/oder ein über den Übertragungskanal (17) übertragenes Signal (TS) mittels einer dem Übertragungskanal (17) nachgeordneten Nachverzerrungseinrichtung (19) gemäß einer Nachverzerrungsvorschrift nachverzerrt wird und erst das nachverzerrte Signal (TS') dem Demodulator (15) zugeführt wird und
- wobei die Vorverzerrungsvorschrift und/oder die Nachverzerrungsvorschrift von einer Einstelleinrichtung (20) während des Rotierens der Gantry (3) immer wieder neu eingestellt werden.

2. Betriebsverfahren nach Anspruch 1,
**dadurch gekennzeichnet,**
**dass** die Datenquelle (11) dem Modulator (12) den Datenstrom (DS) als komplexes Signal zuführt und dass der Modulator (12) das Sendesignal (SS) durch eine Quadraturamplitudenmodulation eines Trägersignals (CS) gemäß dem ihm zugeführten komplexen Signal ermittelt.

3. Betriebsverfahren nach Anspruch 1 oder 2,
**dadurch gekennzeichnet,**
**dass** die Vorverzerrungsvorschrift und/oder die Nachverzerrungsvorschrift parametrierbare FIR-Filterungen sind.

4. Betriebsverfahren nach Anspruch 1, 2 oder 3,
**dadurch gekennzeichnet,**
**dass** die Einstelleinrichtung (20) die Vorverzerrungsvorschrift und/oder die Nachverzerrungsvorschrift in Abhängigkeit von einem jeweiligen Drehwinkel (φ) der Gantry (3) ermittelt.

5. Betriebsverfahren nach Anspruch 4,
**dadurch gekennzeichnet,**
**dass** die Einstelleinrichtung (20) im Rahmen der Ermittlung der Vorverzerrungsvorschrift und/oder der Nachverzerrungsvorschrift zusätzlich zu dem jeweiligen Drehwinkel (φ) mindestens eine zeitliche Ableitung des Drehwinkels (φ) berücksichtigt.

6. Betriebsverfahren nach Anspruch 4 oder 5,
**dadurch gekennzeichnet,**
**dass** der Drehwinkel (φ) jeweils gemessen wird oder dass der Drehwinkel (φ) nur bei vorbestimmten Winkelpositionen gemessen wird und zwischen den vorbestimmten Winkelpositionen durch Fortschreibung des zuletzt gemessenen Drehwinkels (φ) anhand von die Drehung der Gantry (3) charakterisierenden Betriebsdaten (ω, α) der Gantry (3), insbesondere der Drehzahl (ω) und/oder der Drehbeschleunigung (α), ermittelt wird.

7. Betriebsverfahren nach einem der obigen Ansprüche,
**dadurch gekennzeichnet,**
**dass** in der Einstelleinrichtung (20) in einer Lookup-Table (21) für eine Vielzahl von Eingangsgrößen jeweils Parameter für die Parametrierung der Vorverzerrungseinrichtung (18) und/oder der Nachverzerrungseinrichtung (19) hinterlegt sind und dass die Einstelleinrichtung (20) die Parameter unter Verwertung der Lookup-Table (21) ermittelt.

8. Betriebsverfahren nach einem der obigen Ansprüche,
**dadurch gekennzeichnet,**
**dass** der Datenstrom (DS) eine Vielzahl von Datengruppen (26) umfasst, dass die Datengruppen (26) jeweils eine Referenzsequenz (27) und eine Nutzsequenz (28) umfassen, so dass das über den Übertragungskanal (17) übertragene Signal (TS, TS') einen Referenzanteil (30) und einen Nutzanteil (31) umfasst, dass die Referenzanteile (30) der Einstelleinrichtung (20) zugeführt werden und dass die Einstelleinrichtung (20) die Nachverzerrungsvorschrift für den auf den jeweiligen Referenzanteil (30) folgenden Nutzanteil (31) in Abhängigkeit von dem jeweiligen Referenzanteil (30) nachführt.

9. Computertomograph,
- wobei der Computertomograph einen feststehenden Grundkörper (2) und eine relativ zu dem feststehenden Grundkörper (2) rotierbare Gantry (3) aufweist,
- wobei der Computertomograph eine Datenquelle (11), einen Modulator (12), einen Übertragungskanal (17), einen Demodulator (15) und eine Datensinke (16) aufweist, so dass während des Rotierens der Gantry (3) zwischen der Gantry (3) und dem Grundkörper (2) digitale Daten von der Datenquelle (11) über den Modulator (12), den Übertragungskanal (17) und den Demodulator (15) zu der Datensinke (16) übertragbar sind,
- wobei dem Übertragungskanal (17) eine Vorverzerrungseinrichtung (18) vorgeordnet ist, mittels derer ein von dem Modulator (12) durch Modulieren eines dem Modulator (12) von der Datenquelle (11) zugeführten Datenstroms (DS) generiertes Sendesignal (SS) gemäß einer Vorverzerrungsvorschrift vorverzerrt wird, so dass erst das vorverzerrte Sendesignal (SS') dem Übertragungskanal (17) zugeführt wird, und/oder dem Übertragungskanal (17) eine Nachverzerrungseinrichtung (19) nachgeordnet ist, mittels derer ein über den Übertragungskanal (17) übertragenes Signal (TS) gemäß einer Nachverzerrungsvorschrift nachverzerrt wird, so dass erst das nachverzerrte Signal (TS') dem Demodulator (15) zugeführt wird, und
- wobei der Computertomograph eine Einstelleinrichtung (20) aufweist, von der die Vorverzerrungsvorschrift und/oder die Nachverzerrungvorschrift während des Rotierens der Gantry (3) immer wieder neu eingestellt werden.

10. Computertomograph nach Anspruch 9,
**dadurch gekennzeichnet,**
**dass** der Modulator (12) als Quadraturamplitudenmodulator ausgebildet ist, der das Sendesignal (SS) anhand eines ihm von der Datenquelle (11) zugeführten komplexen Signals gemäß einer Quadraturamplitudenmodulation ermittelt.

11. Computertomograph nach Anspruch 9 oder 10,
**dadurch gekennzeichnet,**
**dass** die Vorverzerrungseinrichtung (18) und/oder die Nachverzerrungseinrichtung (19) als parametrierbare FIR-Filter ausgebildet sind.

12. Computertomograph nach Anspruch 9, 10 oder 11,
**dadurch gekennzeichnet,**
**dass** die Einstelleinrichtung (20) die Vorverzerrungsvorschrift und/oder die Nachverzerrungsvorschrift in Abhängigkeit von einem jeweiligen Drehwinkel (φ) der Gantry (3) ermittelt.

13. Computertomograph nach Anspruch 12,
**dadurch gekennzeichnet,**
**dass** die Einstelleinrichtung (20) im Rahmen der Ermittlung der Vorverzerrungsvorschrift und/oder der Nachverzerrungsvorschrift zusätzlich zu dem jeweiligen Drehwinkel (φ) mindestens eine zeitliche Ableitung des Drehwinkels berücksichtigt.

14. Computertomograph nach einem der Ansprüche 9 bis 13,
**dadurch gekennzeichnet,**
**dass** die Einstelleinrichtung (20) eine Lookup-Table (21) aufweist, in der für eine Vielzahl von Eingangsgrößen jeweils Parameter für die Parametrierung der Vorverzerrungseinrichtung (18) und/oder der Nachverzerrungseinrichtung (19) hinterlegt sind, und dass die Einstelleinrichtung (20) die Parameter unter Verwertung der Lookup-Table (21) ermittelt.

15. Computertomograph nach einem der Ansprüche 9 bis 14,
**dadurch gekennzeichnet,**
**dass** der Datenstrom (DS) eine Vielzahl von Datengruppen (26) umfasst, dass die Datengruppen (26) jeweils eine Referenzsequenz (27) und eine Nutzsequenz (28) umfassen, so dass das über den Übertragungskanal (17) übertragene Signal (TS, TS') einen Referenzanteil (30) und einen Nutzanteil (31) umfasst, dass die Referenzanteile (30) der Einstelleinrichtung (20) zugeführt werden und dass die Einstelleinrichtung (20) die Nachverzerrungsvorschrift für den auf den jeweiligen Referenzanteil (30) folgenden Nutzanteil (31) in Abhängigkeit von dem jeweiligen Referenzanteil (30) nachführt.

## Claims

1. Operating method for a computed tomography system,
- wherein a gantry (3) of the computed tomography system is rotated relative to a fixed base body (2) of the computed tomography system,
- wherein during the rotation of the gantry (3) between the gantry (3) and the base body (2) digital data is transmitted from a data source (11) via a modulator (12), a transmission channel (17) and a demodulator (15) to a data sink (16),
- wherein a transmission signal (SS) generated by the modulator (12) by modulating a data stream (DS) supplied to the modulator (12) from the data source (11) is pre-distorted by means of a pre-distortion facility (18) arranged upstream of the transmission channel (17) in accordance with a pre-distortion rule and only the pre-distorted transmission signal (SS') is supplied to the transmission channel (17) and/or a transmitted signal (TS) transmitted via the transmission channel (17) is post-distorted by means of a post-distortion facility (19) arranged downstream of the transmission channel (17) in accordance with a post-distortion rule and only the post-distorted signal (TS') is supplied to the demodulator (15) and
- wherein the pre-distortion rule and/or the post-distortion rule are repeatedly reset by a setting facility (20) during the rotation of the gantry (3).

2. Operating method according to claim 1,
**characterised in that**
the data source (11) supplies the data stream (DS) to the modulator (12) as a complex signal, and **in that** the modulator (12) determines the transmission signal (SS) by a quadrature amplitude modulation of a carrier signal (CS) in accordance with the complex signal supplied to it.

3. Operating method according to claim 1 or 2,
**characterised in that**
the pre-distortion rule and/or the post-distortion rule are parameterisable FIR filters.

4. Operating method according to claim 1, 2 or 3,
**characterised in that**
the setting facility (20) determines the pre-distortion rule and/or the post-distortion rule as a function of a respective angle of rotation (φ) of the gantry (3).

5. Operating method according to claim 4,
**characterised in that**
the setting facility (20) takes into account at least one time derivative of the angle of rotation (φ) in addition to the respective angle of rotation (φ) when determining the pre-distortion rule and/or the post-distortion rule.

6. Operating method according to claim 4 or 5,
**characterised in that**
the angle of rotation (φ) is measured in each case or that the angle of rotation (φ) is measured only at predetermined angular positions and is determined between the predetermined angular positions by updating the last measured angle of rotation (φ) on the basis of operating data (ω, α) of the gantry (3) characterising the rotation of the gantry (3), in particular the rotational speed (ω) and/or the rotational acceleration (α).

7. Operating method according to one of the preceding claims,
**characterised in that**
the parameters for the parameterisation of the pre-distortion facility (18) and/or the post-distortion facility (19) are stored in a lookup table (21) in the setting facility (20) for a multiplicity of input variables, and **in that** the setting facility (20) determines the parameters using the lookup table (21).

8. Operating method according to one of the preceding claims,
**characterised in that**
the data stream (DS) comprises a multiplicity of data groups (26), **in that** the data groups (26) each comprise a reference sequence (27) and a useful sequence (28), so that the transmitted signal (TS, TS') transmitted via the transmission channel (17) comprises a reference portion (30) and a useful portion (31), **in that** the reference portions (30) are supplied to the setting facility (20) and **in that** the setting facility (20) tracks the post-distortion rule for the useful portion (31) following the respective reference portion (30) as a function of the respective reference portion (30).

9. Computed tomography system,
- wherein the computed tomography system comprises a fixed base body (2) and a gantry (3) which can be rotated relative to the fixed base body(2),
- wherein the computed tomography system comprises a data source (11), a modulator (12), a transmission channel (17), a demodulator (15) and a data sink (16), so that during the rotation of the gantry (3) between the gantry (3) and the base body (2) digital data is transmitted from the data source (11) via the modulator (12), the transmission channel (17) and the demodulator (15) to the data sink (16),
- wherein arranged upstream of the transmission channel (17) there is a pre-distortion facility (18), by means of which a transmission signal (SS) generated by the modulator (12) by modulating a data stream (DS) supplied to the modulator (12) from the data source (11) is pre-distorted in accordance with a pre-distortion rule, so that only the pre-distorted transmission signal (SS') is supplied to the transmission channel (17), and/or a post-distortion facility (19) is arranged downstream of the transmission channel (17) by means of which a transmitted signal (TS) transmitted via the transmission channel (17) is post-distorted in accordance with a post-distortion rule, so that only the post-distorted signal (TS') is supplied to the demodulator (15), and
- wherein the computed tomography system has a setting facility (20) by which the pre-distortion rule and/or the post-distortion rule are repeatedly reset during the rotation of the gantry (3).

10. Computed tomography system according to claim 9,
**characterised in that**
the modulator (12) is designed as a quadrature amplitude modulator which determines the transmission signal (SS) on the basis of a complex signal supplied to it from the data source (11) in accordance with quadrature amplitude modulation.

11. Computed tomography system according to claim 9 or 10,
**characterised in that**
the pre-distortion facility (18) and/or the post-distortion facility (19) are designed as parameterisable FIR filters.

12. Computed tomography system according to claim 9, 10 or 11,
**characterised in that**
the setting facility (20) determines the pre-distortion rule and/or the post-distortion rule as a function of a respective angle of rotation (φ) of the gantry (3).

13. Computed tomography system according to claim 12,
**characterised in that**
the setting facility (20) takes into account at least one time derivative of the angle of rotation in addition to the respective angle of rotation (φ) when determining the pre-distortion rule and/or the post-distortion rule.

14. Computed tomography system according to one of claims 9 to 13,
**characterised in that**
the setting facility (20) has a lookup table (21) in which parameters for parameterising the pre-distortion facility (18) and/or the post-distortion facility (19) are stored for a multiplicity of input variables in each case, and **in that** the setting facility (20) determines the parameters using the lookup table (21).

15. Computed tomography system according to one of claims 9 to 14,
**characterised in that**
the data stream (DS) comprises a multiplicity of data groups (26), **in that** the data groups (26) each comprise a reference sequence (27) and a useful sequence (28), so that the transmitted signal (TS, TS') transmitted via the transmission channel (17) comprises a reference portion (30) and a useful portion (31), **in that** the reference portions (30) are supplied to the setting facility (20) and **in that** the setting facility (20) tracks the post-distortion rule for the useful portion (31) following the respective reference portion (30) as a function of the respective reference portion (30).

## Revendications

1. Procédé pour faire fonctionner un tomodensitomètre assisté par ordinateur,
- dans lequel on fait tourner un portique (3) du tomodensitomètre assisté par ordinateur par rapport à une embase (2) fixe du tomodensitomètre assisté par ordinateur,
- dans lequel, pendant la rotation du portique (3), on transmet, entre le portique (3) et l'embase (2), des données numériques d'une source (11) de données à un puits (16) de données, en passant par un modulateur (12), un canal (17) de transmission et un démodulateur (15),
- dans lequel on distord antérieurement, suivant une prescription de distorsion antérieure, au moyen d'un dispositif (18) de distorsion antérieure monté en amont du canal (17) de transmission, un signal (SS) d'émission créé par le modulateur (12) par modulation d'un flux (DS) de données envoyé au modulateur (12) par la source (11) de données et on envoie au canal (17) de transmission seulement le signal (SS') d'émission distordu antérieurement et/ou on distord postérieurement, suivant une prescription de distorsion postérieure, au moyen d'un dispositif (19) de distorsion postérieure monté en aval du canal (17) de transmission, un signal (TS) transmis en passant par le canal (17) de transmission et on envoie au démodulateur (15) seulement le signal (TS') distordu postérieurement, et
- dans lequel on rerègle toujours à nouveau la prescription de distorsion antérieure et/ou la prescription de distorsion postérieure par un dispositif (20) de réglage pendant la rotation du portique (3).

2. Procédé suivant la revendication 1,
**caractérisé**
**en ce que** la source (11) de données envoie au modulateur (12) le flux (DS) de données sous la forme d'un signal complexe et **en ce que** le modulateur (12) détermine le signal (SS) d'envoi par une modulation d'amplitude en quadrature d'un signal (CS) porteur conformément au signal complexe, qui lui est envoyé.

3. Procédé suivant la revendication 1 ou 2,
**caractérisé**
**en ce que** la prescription de distorsion antérieure et/ou la prescription de distorsion postérieure sont des filtrages FIR paramétrables.

4. Procédé suivant la revendication 1, 2 ou 3,
**caractérisé**
**en ce que** le dispositif (20) de réglage détermine la prescription de distorsion antérieure et/ou la prescription de distorsion postérieure en fonction d'un angle (φ) de rotation respectif du portique (3).

5. Procédé suivant la revendication 4,
**caractérisé**
**en ce que** le dispositif (20) de réglage tient compte, dans le cadre de la détermination de la prescription de distorsion antérieure et/ou de la prescription de distorsion postérieure, en plus de l'angle (φ) de rotation respectif, au moins d'une dérivée en fonction du temps de l'angle (φ) de rotation.

6. Procédé suivant la revendication 4 ou 5,
**caractérisé**
**en ce que** l'on mesure, entre les positions angulaires déterminées à l'avance, en continuant à prendre en compte l'angle (φ) de rotation mesuré en dernier, respectivement l'angle (φ) de rotation ou **en ce que** l'on mesure l'angle (φ) de rotation seulement en des positions angulaires déterminées à l'avance et on détermine des données (ω, α) de fonctionnement du portique (3) caractérisant la rotation du portique (3), en particulier la vitesse (ω) ou l'accélération (α) de rotation.

7. Procédé suivant l'une des revendications précédentes,
**caractérisé**
**en ce que** l'on met en mémoire respectivement des paramètres, pour le paramétrage du dispositif (18) de distorsion antérieure et/ou du dispositif (19) de distorsion postérieure, dans le dispositif (20) de réglage dans une table (21) de consultation d'une pluralité de grandeurs d'entrée et **en ce que** le dispositif (20) de réglage détermine les paramètres en tirant parti de la table (21) de consultation.

8. Procédé suivant l'une des revendications précédentes,
**caractérisé**
**en ce que** le flux (DS) de données comprend une pluralité de groupes (26) de données, **en ce que** les groupes (26) de données comprennent chacun une séquence (27) de référence et une séquence (28) utile, de manière à ce qu'un signal (TS, TS') transmis en passant par le canal (17) de transmission comprenne une partie (30) de référence et une partie (31) utile, **en ce que** l'on envoie les parties (30) de référence au dispositif (20) de réglage et **en ce que** le dispositif (20) de réglage suit, en fonction de la partie (30) de référence respective, la prescription de distorsion postérieure pour la partie (31) utile suivant la partie (30) de référence respective.

9. Tomodensitomètre assisté par ordinateur,
- dans lequel le tomodensitomètre assisté par ordinateur a une embase (2) fixe et un portique (3) tournant par rapport à l'embase (2) fixe,
- dans lequel le tomodensitomètre assisté par ordinateur a une source (11) de données, un modulateur (12), un canal (17) de transmission, un démodulateur (15) et un puits (16) de données, de manière à pouvoir transmettre de la source (11) de données au puits (16) de données en passant par le modulateur (12), le canal (17) de transmission et le démodulateur (15), des données numériques entre le portique (3) et l'embase (2) pendant la rotation du portique (3),
- dans lequel il est monté, en amont du canal (17) de transmission, un dispositif (18) de distorsion antérieure, au moyen duquel on distord, suivant une prescription de distorsion antérieure, un signal (SS) d'envoi créé par le modulateur (12) par modulation d'un flux (DS) de données envoyé au modulateur (12) par la source (11) de données, de manière à envoyer au canal (17) de transmission seulement le signal (SS') d'envoi distordu antérieurement et/ou il est monté, en aval du canal (17) de transmission, un dispositif (19) de distorsion postérieure, au moyen duquel un signal (TS) transmis en passant par le canal (17) de transmission est distordu postérieurement suivant une prescription de distorsion postérieure, de manière à envoyer au démodulateur (15) seulement le signal (TS') distordu postérieurement, et
- dans lequel le tomodensitomètre assisté par ordinateur a un dispositif (20) de réglage, par lequel la prescription de distorsion antérieure et/ou la prescription de distorsion postérieure sont toujours reréglées à nouveau pendant la rotation du portique (3).

10. Tomodensitomètre assisté par ordinateur suivant la revendication 9,
**caractérisé**
**en ce que** le modulateur (12) est constitué sous la forme d'un modulateur d'amplitude en quadrature, qui détermine, suivant une modulation en amplitude en quadrature, le signal (SS) envoyé à l'aide d'un signal complexe, qui lui est envoyé par la source (11) de données.

11. Tomodensitomètre assisté par ordinateur suivant la revendication 9 ou 10,
**caractérisé**
**en ce que** le dispositif (18) de distorsion antérieure et/ou le dispositif (19) de distorsion postérieure sont des filtres FIR paramétrables.

12. Tomodensitomètre assisté par ordinateur suivant la revendication 9, 10 ou 11,
**caractérisé**
**en ce que** le dispositif (20) de réglage détermine la prescription de distorsion antérieure et/ou la prescription de distorsion postérieure en fonction d'un angle (φ) de rotation respectif du portique (3).

13. Tomodensitomètre assisté par ordinateur suivant la revendication 12,
**caractérisé**
**en ce que** le dispositif (20) de réglage tient compte, dans le cadre de la détermination de la prescription de distorsion antérieure et/ou de la prescription de distorsion postérieure, en plus de l'angle (φ) de rotation respectif, au moins d'une dérivée en fonction du temps de l'angle de rotation.

14. Tomodensitomètre assisté par ordinateur suivant l'une des revendications 9 à 13,
**caractérisé**
**en ce que** le dispositif (20) de réglage a une table (21) de consultation, dans laquelle sont mis en mémoire, pour une pluralité de grandeurs d'entrée, respectivement des paramètres pour le paramétrage du dispositif (18) de distorsion antérieure et/ou le dispositif (19) de distorsion postérieure, et **en ce que** le dispositif (20) de réglage détermine les paramètres en tirant parti de la table (21) de consultation.

15. Tomodensitomètre assisté par ordinateur suivant l'une des revendications 9 à 14,
**caractérisé**
**en ce que** le flux (DS) de données comprend une pluralité de groupes (26) de données, **en ce que** les groupes (26) de données comprennent chacun une séquence (27) de référence et une séquence (28) utile, de manière à ce qu'un signal (TS, TS') transmis en passant par le canal (17) de transmission comprenne une partie (30) de référence et une partie (31) utile, **en ce que** l'on envoie les parties (30) de référence au dispositif (20) de réglage et **en ce que** le dispositif (20) de réglage suit, en fonction de la partie (30) de référence respective, la prescription de distorsion postérieure pour la partie (31) utile suivant la partie (30) de référence respective.
